**Europäisches Patentamt**

**European Patent Office**

**Office européen des brevets**

(19)

(11) Veröffentlichungsnummer: **0 126 100**
**B1**

(12) # EUROPÄISCHE PATENTSCHRIFT

(45) Veröffentlichungstag der Patentschrift:
**05.10.88**

(51) Int. Cl.⁴: **G 01 N 33/28, G 01 N 27/18**

(21) Anmeldenummer: **83903515.1**

(22) Anmeldetag: **11.11.83**

(86) Internationale Anmeldenummer:
**PCT/EP 83/00297**

(87) Internationale Veröffentlichungsnummer:
**WO 84/02003 (24.05.84 Gazette 84/13)**

(54) **VERFAHREN UND VORRICHTUNG ZUR BESTIMMUNG DES ERREICHENS ODER ÜBERSCHREITENS EINES PROZENTUALEN GRENZWERTES EINES IN EINEM FLÜSSIGKEITSGEMISCH ENTHALTENEN FLÜSSIGKEITSANTEILES NIEDRIGERER VERDAMPFUNGSTEMPERATUR.**

(30) Priorität: **18.11.82 DE 3242506**

(43) Veröffentlichungstag der Anmeldung:
**28.11.84 Patentblatt 84/48**

(45) Bekanntmachung des Hinweises auf die Patenterteilung:
**05.10.88 Patentblatt 88/40**

(84) Benannte Vertragsstaaten:
**BE CH DE FR GB LI NL SE**

(56) Entgegenhaltungen:
**EP - A - 0 056 424**
**EP - A - 0 070 801**
**FR - A - 2 009 392**
**FR - A - 2 193 487**
**FR - A - 2 374 638**
**GB - A - 2 042 737**
**US - A - 3 780 565**
**US - A - 4 106 331**

(73) Patentinhaber: **COLLINS, Hans-Jürgen, Im Sieke 45, D-3300 Braunschweig (DE)**

(72) Erfinder: **LHOTZKY, Kurt, Heimgarten 17A, D-3300 Braunschweig (DE)**

(74) Vertreter: **Döring, Rudolf, Dr.-Ing., Patentanwälte Dr.-Ing. R. Döring Dipl.-Phys. Dr. J. Fricke Jasperallee 1a, D-3300 Braunschweig (DE)**

**Beschreibung**

Die Erfindung betrifft ein Verfahren zur Bestimmung des Erreichens oder Überschreitens eines vorgegebenen prozentualen Grenzwertes des in einem Flüssigkeitsgemisch enthaltenen Anteils mit einer niedrigeren Verdampfungstemperatur als die übrige Flüssigkeit insbesondere des Wassers in einer Bremsflüssigkeit, mit Hilfe eines elektrischen Weiderstandsdrahtes mit positivem Temperaturkoeffizienten, welcher in das Flüssigkeitsgemisch eingetaucht sowie mit einer solchen elektrischen Energiemenge beaufschlagt wird, bei welcher sich die Energieumwandlung in der Probe merkbar verändert, und durch dessen Widerstandsänderung eine Messgrösse beeinflusst wird. Ferner bezieht sich die Erfindung auf eine Vorrichtung zur Durchführung des vorgenannten Verfahrens.

Es sind Verfahren und Vorrichtungen, insbesondere für die Feststellung eines prozentualen Grenzwertes des Wassers in einer Bremsflüssigkeit, bekannt, welche von der Erkenntnis ausgehen, dass mit zunehmendem Wassergehalt in der Bremsflüssigkeit der Siedepunkt dieser Flüssigkeit bzw. des Gemisches herabgesetzt wird und somit ein Mass für den Anteil des Wassers in der Bremsflüssigkeit darstellt.

Bei einem bekannten Verfahren dieser Art wird jeweils eine Probemenge der Flüssigkeit entnommen und in einem Prüfraum bis zur Dampfbildung erhitzt. Dabei erfolgt eine Temperaturmessung und eine Füllungsüberwachung, um bei einer Verdrängung der Flüssigkeit durch die Dampfbildung aus dem Prüfraum den zugeordneten Temperaturmesswert als Mass für den Siedepunkt der Flüssigkeit zu ermitteln (DE-A-27 21 232).

Bei einem anderen Verfahren (EP A-0 056 424) wird mittels einer Messsonde ebenfalls eine Probe der Flüssigkeit entnommen und erhitzt, wobei die Temperatur und die zeitliche Temperaturänderung des in die Probe hineinragenden Heizelementes gemessen wird und bei einer Änderung der zeitlichen Temperaturänderung die erreichte Temperatur registriert wird als Mass für den Siedepunkt der Bremsflüssigkeit.

Die vorgenannten Verfahren erfordern für jede Messung die Entnahme einer Probemenge, welche von der Hauptmenge des Flüssigkeitsgemisches separiert und nach der Messung wieder der Hauptmenge zugeführt werden muss. Das für jede Messung notwendige Abtrennen einer Probemenge sowie deren Überführung in einen Druckraum und ihre Erwärmung bis zur Bildung eines Dampfvolumens in dem Druckraum ist aufwendig sowie verhältnismässig kompliziert und erfordert eine relativ lange Zeitspanne für die Erwärmung und Abkühlung der jeweils entnommenen Probemenge.

Bei einem weiteren bekannten Verfahren der einleitend genannten Art (GB-A-20 42 737) wird die Entnahme einer Probemenge vermieden und für die Ermittlung des Wassergehaltes in einem insbesondere zu Härtezwecken dienenden Ölbad ein elektrischer temperaturabhängiger Widerstand in das Bad eingetaucht sowie mit soviel Energie gespeist, dass er eine konstante Temperatur oberhalb der Verdampfungstemperatur des Wassers annimmt. Wenn der Widerstand abkühlt und sein Widerstandswert als Ergebnis der Wasserverdampfung sinkt, wird ein Signal ausgelöst und veranlasst, dass die Energiezufuhr erhöht wird, bis der Widerstand seine vorgegebene Temperatur wieder erreicht. Die Impulse der erhöhten Energiezufuhr werden erfasst und lösen ein Signal aus, wenn soviel Wasser in dem Öl ist, dass der zeitliche Änderungswert der Impulse einen vorgegebenen Durchschnittswert überschreitet.

Das vorgenannte Verfahren erfordert für die Aufrechterhaltung der konstanten Temperatur des elektrischen Widerstandes oberhalb der Verdampfungstemperatur des Wassers über die gesamte Dauer der Messung eine entsprechende Energieeinspeisung in den temperaturabhängigen Widerstand, wobei für die Erzielung und Aufrechterhaltung der stationären Verhältnisse während der Messung relativ lange Einspeisezeiten der Energie in den Widerstand notwendig sind. Hierdurch erfolgt eine zusätzliche Erwärmung und Beeinflussung des Zustandes des Flüssigkeitsgemisches, die sich besonders bei temperaturempfindlichen Flüssigkeiten und auch bei kleineren Flüssigkeitsmengen sehr nachteilig bemerkbar machen.

Bei einem weiteren bekannten Verfahren (FR-A-20 09 392) zur Messung der Wärmeleitfähigkeit von Gasen und Flüssigkeiten wird ein Hitzdraht durch Stromimpulse auf eine bestimmte Temperatur aufgeheizt und in den jeweiligen Zeitintervallen zwischen zwei Stromimpulsen der Abkühlvorgang des Drahtes elektronisch gemessen und registriert. Der Hitzdraht wird dabei während fester in kurzer Folge mit dazwischenliegenden Abkühlphasen über feste Zeitintervalle mit den Stromimpulsen beaufschlagt. Für die Messung wird die Erkenntnis ausgenutzt, dass bei einem plötzlichen Stromabfall der Wiederstandswert eines Hitzdrahtes sich entlang einer Widerstandskennlinie für einen idealen Widerstand, d.h. für einen Widerstand entsprechend dem Ohmschen Gesetz, ändert, um aus den Unterschiedswerten zwischen Punkten dieser Kennlinie und den diesen entsprechenden Punkten der tatsächlichen Kennlinie die Messwerte für die Bestimmung der Leitfähigkeit des den Hitzdraht umgebenden Mediums zu gewinnen.

Durch die Folge der Stromimpulse erfolgt auch hier eine Aufheizung des den Hitzdraht umgebenden Mediums. Ein Kriterium für den in einem Flüssigkeitsgemisch enthaltenen Anteil eines Bestandteiles mit niedrigerer Verdampfungstemperatur liefert dieses Verfahren nicht.

Um Fluide, die sich schwierig z.B. von Wasser unterscheiden lassen, feststellen zu können, ist es weiterhin bekannt geworden (FR-A-23 74 638) einen temperaturabhängigen Widerstand in das Fluidgemisch einzubringen und auf konstanter Temperatur zu halten sowie den Leistungsfluss zu messen und anzuzeigen. Dabei wird der Leistungsfluss als Mass für die Wärmeaufnahmeei-

genschaften des umgebenden Mediums ausgewertet. Dabei werden Referenzwerte des einen in das bekannte Medium eingetauchten Widerstandes mit den Werten des anderen in das Gemisch eintauchenden Widerstandes in Vergleich gesetzt.

Auch dieses bekannte Verfahren ist für temperaturempfindliche Flüssigkeiten und für Messungen in kleineren Flüssigkeitsmengen nicht geeignet, abgesehen davon, dass Grenzwerte des Anteiles der unbekannten Flüssigkeit mit dem bekannten Verfahren nicht ohne weiteres festgestellt werden können.

Aufgabe vorliegender Erfindung ist es, das Verfahren der eingangs genannten Art so auszubilden, dass eine sehr kurzzeitige, die Temperatur des Flüssigkeitsgemisches praktisch nicht beeinträchtigende Energieeinspeisung in den Widerstandsdraht für die Messung ausreicht und eine kurzfristige Folge der Messungen auch in kleinen Mengen des Flüssigkeitsgemisches ermöglicht wird.

Zur Lösung vorstehender Aufgabe kennzeichnet sich das eingangs näher genannte Verfahren erfindungsgemäss durch die im Anspruch 1 oder im Anspruch 2 genannten Merkmale.

Der Erfindung liegt die Erkenntnis zugrunde, dass bei der Erwärmung eines in dem Flüssigkeitsgemisch befindlichen elektrischen Widerstandsdrahtes mit positivem Temperaturkoeffizienten bis über die Verdampfungstemperatur der Flüssigkeitsmischung eine sich nahezu sprunghaft einstellende Widerstandsänderung auftritt mit der Folge, dass

a) bei Zuführung einer vorbestimmten definierten elektrischen Energiemenge eine spontane Änderung der Zeitspanne für die Energieumwandlung messbar ist bzw.

b) bei vorbestimmter definierter Zeitspanne eine spontane Änderung der umgewandelten Energiemenge feststellbar ist.

Da sich die Siedetemperatur des Flüssigkeitsgemisches in Abhängigkeit von dem Anteil der Flüssigkeit mit einer niedrigeren Verdampfungstemperatur ändert, kann durch eine entsprechend vorbestimmbare dem Widerstandsdraht zuzuführende Energiemenge dessen Erwärmung bis über die jeweilige Siedetemperatur erreicht werden.

Ausgehend von diesen Erkenntnissen ist es somit gemäss dem Anspruch 1 lediglich notwendig, in einer Probe des zu untersuchenden Flüssigkeitsgemisches, in der dieser Grenzwert des Flüssigkeitsanteiles mit der niedrigeren Verdampfungstemperatur vorgegeben ist, einen elektrischen Widerstandsdraht mit verschiedenen zugeführten Energiemengen durch Stromimpulse zu erhitzen und die Energiemenge zu ermitteln, bei der sich die Zeitspanne für die Einspeisung sprunghaft ändert und dann den Widerstandsdraht in das zu untersuchende Flüssigkeitsgemisch einzutauchen und mit den in der Probe ermittelten Energiemengen, bei welchen sich die Zeitspanne für die Einspeisung merkbar verändert, zu beaufschlagen und dabei die Zeitspannen mit dem in der Probe bei sprunghafter Änderung der Einspeisezeit ermittelten Wert zu vergleichen. Solange der vorgegebene prozentuale Grenzwert des Anteiles der Flüssigkeit mit niedrigerer Verdampfungstemperatur nicht erreicht ist, werden in dem Flüssigkeitsgemisch Einspeisezeiten gemessen, die merkbar von dem bei sprunghafter Änderung ermittelten Wert der Probe abweichen. Mit Erreichen oder Überschreiten des genannten prozentualen Grenzwertes des Flüssigkeitsanteiles mit niedrigerer Verdampfungstemperatur ergibt sich hingegen eine Energie-Einspeisezeit, die dem bei sprunghafter Änderung des Wertes in der Probe entspricht.

Statt bei vorgegebenen definierten Energiemengen die Zeitspannen für die Energieumwandlung als Messgrösse zu verwenden, kann gemäss Anspruch 2 auch umgekehrt verfahren werden, indem die Zeitspanne für die Zuführung der elektrischen Energie zu dem Widerstandsdraht vorgegeben wird und als Messgrösse die eingespeiste Energiemenge gemessen wird, wobei ebenfalls zunächst in der Probe derjenige Wert ermittelt wird, bei dem sich die eingespeiste Energiemenge sprunghaft ändert, um nachfolgend den Widerstandsdraht in dem Flüssigkeitsgemisch während der gleichen Zeitspanne wie in der Probe mit der dort ermittelten Energiemenge, die zu einer sprunghaften Änderung des Wertes der Energiemenge geführt hat, zu beaufschlagen und die eingespeiste Energiemenge mit der dem Widerstandsdraht in der Probe bei sprunghafter Änderung zugeführten Energiemenge zu vergleichen. Bis zum Erreichen des vorgegebenen prozentualen Grenzwertes des Anteiles der Flüssigkeit mit niedrigerer Verdampfungstemperatur in dem Flüssigkeitsgemisch werden die zu vergleichenden Energiemengen unterschiedlich sein, während sie bei Erreichen oder Überschreiten des vorgenannten prozentualen Grenzwertes des Anteiles der Flüssigkeit mit niedrigerer Verdampfungstemperatur etwa in der gleichen Grösse gemessen werden.

Bei dem neuen Verfahren ist es somit lediglich notwendig, für einen vorgegebenen prozentualen Grenzwert des in einem Flüssigkeitsgemisch enthaltenen Anteiles der Flüssigkeit mit einer niedrigeren Verdampfungstemperatur einmalig eine repräsentative Probe dieses Flüssigkeitsgemisches herzustellen, d.h. eine Probe, in der dieser Grenzwert vorliegt, um die für die späteren Messungen in dem zu untersuchenden Flüssigkeitsgemisch notwendigen Messgrössen bzw. Daten zu ermitteln, und dann eine beliebige Anzahl von Messungen in dem zu untersuchenden Flüssigkeitsgemisch vorzunehmen.

Die Messungen in dem Flüssigkeitsgemisch erfolgen dabei durch unmittelbares Eintauchen des Widerstandsdrahtes in das Flüssigkeitsgemisch, ohne dass durch die Messungen eine merkbare Beeinträchtigung des Flüssigkeitsgemisches hinsichtlich seiner Temperatur, seiner Zusammensetzung oder dgl. erfolgt. Aus diesem Grunde können zahlreiche Messungen kurzfristig hintereinander in dem Flüssigkeitsgemisch vorgenommen werden, was beispielsweise erforderlich ist, wenn durch Zugabe eines Flüssigkeitsanteiles ein bestimmtes Mischungsverhältnis hergestellt und unmittelbar nach Erreichen angezeigt werden soll.

In anderen Fällen, in denen nur eine allmähliche Veränderung des Mischungsverhältnisses erfolgt, beispielsweise bei hygroskopischen Flüssigkeiten, welche Wasser aus der Umgebung aufnehmen, können nach der bereits genannten erstmaligen Ermittlung der entsprechenden Messgrössen bzw. Daten in der repräsentativen Probe die weiteren Messungen in grösseren Zeitabständen vorgenommen werden. Die Messungen in der repräsentativen Probe können dabei im Labor also völlig getrennt von den späteren Messungen in dem Flüssigkeitsgemisch erfolgen.

Als besonders zweckmässig hat es sich erwiesen, wenn dem elektrischen Widerstandsdraht die elektrischen Energiemengen bzw. Stromimpulse in der Form eines Auf- oder Entladestromes eines Kondensators oder Speichers zugeführt werden. Neben dem geringen Aufwand für die vorgenannte Art der Zuführung der Energiemengen zu dem Widerstandsdraht ergibt sich der weitere Vorteil der einfachen Ermittlung der jeweiligen Messgrössen.

Wenn die Zeitspannen der Energieumwandlung als Messgrösse gemäss dem Verfahren nach Anspruch 1 vorgesehen sind, empfiehlt es sich, bei der Zuführung von elektrischen Energiemengen durch Kondensatorentladungen als Messgrösse die Zeitspannen zwischen etwa dem Maximum und etwa 20% des Maximums der Spannung an dem Widerstandsdraht zu messen. Dieses Verfahren, bei dem mit Kondensatorentladungen und der vorgenannten Zeitmessung gearbeitet wird, hat sich bereits in der Praxis bewährt und zeichnet sich dadurch aus, dass es nur geringe Aufwendungen erfordert und zu sehr signifikanten sowie gut reproduzierbaren Ergebnissen führt.

Insbesondere bei Messungen mit grösserem zeitlichem Abstand können sich Fehler durch Abweichungen der einspeisbaren Energiemengen, z.B. durch Änderungen der Kapazität oder Spannung der Energiequelle ergeben. Um hier Abhilfe zu schaffen, ist bei einer zweckmässigen Weiterbildung des Verfahrens vorgesehen, dass zur Kompensation von Abweichungen der einspeisbaren Energiemengen, durch Änderungen der Daten der Energiequelle, wie Kapazitätsänderungen oder Spannungsschwankungen, die jeweils für die Messungen in dem Flüssigkeitsgemisch vorgesehenen Energiemengen einem Konstantwiderstand zugeführt werden und die für die Untersuchung des Flüssigkeitsgemisches massgebliche Messgrösse als Ist-Grösse ermittelt und mit der bei der Soll-Menge der eingespeisten Energiemenge ermittelten Messgrösse verglichen wird, und dass die bei der Messung in dem Flüssigkeitsgemisch ermittelte Messgrösse im Verhältnis des Ist-Wertes zum Soll-Wert der Messgrösse bei Beaufschlagung des Konstantwiderstandes korrigiert wird.

Eine exakte Messung erfordert weiterhin, dass die durch Temperaturänderungen des Flüssigkeitsgemisches bedingten Abweichungen der Messgrössen ebenfalls berücksichtigt werden.

Dies ist von besonderer Bedeutung bei Flüssigkeitsgemischen, die grösseren Temperaturschwankungen unterworfen sind, wie Hydraulikflüssigkeiten und dgl. Aus dem vorstehenden Grunde ist gemäss einer weiteren Ausbildung des Verfahrens vorgesehen, dass zur Berücksichtigung der Temperaturänderungen des zu untersuchenden Flüssigkeitsgemisches ein Widerstandsdraht mit den gleichen elektrischen und mechanischen Daten wie der in das Flüssigkeitsgemisch eintauchbare Draht in eine Referenzflüssigkeit eingebracht wird, welche nicht die merkbaren Änderungen der Messgrössen des Gemisches aufweist, dass dem Widerstandsdraht in der Referenzflüssigkeit die jeweils für die Messungen in dem Flüssigkeitsgemisch vorgesehenen Energiemengen bzw. Stromimpulse bei der jeweiligen Betriebstemperatur und bei der Ausgangstemperatur des Flüssigkeitsgemisches zugeführt und die für die Messung im Flüssigkeitsgemisch vorgesehenen Messgrössen ermittelt werden, und dass im Verhältnis dieser Werte der Ist-Wert der in dem Flüssigkeitsgemisch ermittelten Messgrösse korrigiert wird.

Eine für die Durchführung der erstbeschriebenen Variante des Verfahrens geeignete Vorrichtung, bei der also als Messgrösse die Zeitspannen für die Energieumwandlung der dem elektrischen Widerstandsdraht zugeführten elektrischen Energie vorgesehen ist, ergibt sich aus dem Anspruch 7.

Durch die beschriebene Verwendung eines elektrischen Kondensators als Energiequelle ergibt sich ein besonders einfacher Aufbau der Vorrichtung, wobei man durch Änderung der Ladespannung des Kondensators in sehr einfacher Weise die dem Widerstandsdraht zuzuführenden Energiemengen einstellen kann. Der Kondensator dient dabei gleichzeitig als Energiespeicher und auch als Teil des Messkreises, welcher mit der Messelektronik verbunden ist, um die Entladezeiten zu messen. Dabei werden zweckmässigerweise nur diejenigen Zeiten erfasst, die zwischen dem Spannungsmaximum und etwa 20% des Maximums der Spannung an dem Widerstandsdraht vergehen.

Für die Durchführung des Verfahrens nach der anderen Variante gemäss dem Anspruch 2 empfiehlt sich die Ausbildung der Vorrichtung entsprechend dem Anspruch 8.

Statt der vorgenannten Verwendung eines elektrischen Kondensators oder Speichers kann bei einer anderen Ausführung der Vorrichtung zur Durchführung des Verfahrens auch eine elektrische Batterie vorgesehen sein, wie dies im Anspruch 9 zum Ausdruck gebracht ist.

Die obengenannten Vorrichtungen können unabhängig davon, ob sie als Messgrössen die Energiemengen oder die Zeitspannen anzeigen zur Berücksichtigung von Schwankungen der einspeisbaren Energiemengen oder von Temperaturschwankungen gemäss den Ansprüchen 10 und 11 weiter- bzw. ausgebildet sein.

Die in der Zeichnung wiedergegebenen Darstellungen zeigen folgendes:

Fig. 1 in schematischer Darstellung ein Ausführungsbeispiel zur Durchführung des Verfahrens,

Fig. 2 die graphische Darstellung eines Messergebnisses, welches mittels des neuen Verfahrens ermittelt wurde,

Fig. 3 das Schaltschema für eine Vorrichtung zur Durchführung des Verfahrens mit einer Batterie als Energiespeicher.

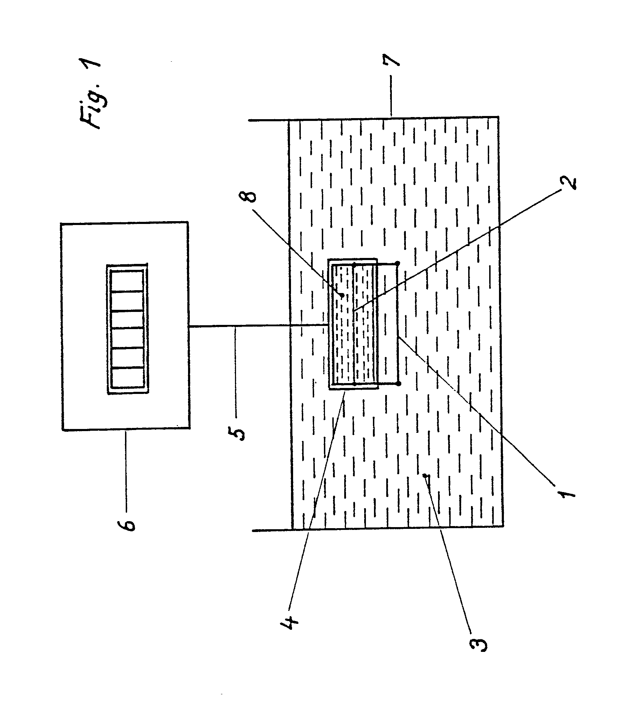

Gemäss Fig. 1 befindet sich in einem schematisch wiedergegebenen Behälter 7 die Flüssigkeitsmischung 3, welche untersucht werden soll. In die Flüssigkeitsmischung eingetaucht ist ein gespannt gehaltener elektrischer Widerstandsdraht 1 mit positivem Temperaturkoeffizienten, wie beispielsweise ein Wolframdraht. Dieser Draht 1 ist an einer Ampulle 4 befestigt und über ein Verbindungskabel 5 mit einer Messelektronik und Anzeigevorrichtung 6 verbunden. Die Ampulle 4 ist mit einer Flüssigkeit 8 gefüllt, deren Siedepunkt höher liegt als der Siedepunkt des Flüssigkeitsmisches 3. In der Ampulle erkennt man einen weiteren Widerstandsdraht 2, welcher hinsichtlich seiner mechanischen Abmessungen und elektrischen Eigenschaften dem Widerstandsdraht 1 entspricht. Der Widerstandsdraht 2 ist ebenfalls über das Verbindungskabel 5 mit der Messelektronik und Anzeigevorrichtung 6 verbunden.

Die nachstehende Beschreibung gibt die beispielsweise Verwendung der Anordnung nach Fig. 1 gemäss der Verfahrensvariante 1 wieder:

Die Messelektronik und Anzeigevorrichtung 6 ist so ausgebildet, dass wahlweise ein in dieser Vorrichtung befindlicher aufladbarer Kondensator über den Widerstandsdraht 2 oder den Widerstandsdraht 1 entladen werden kann. Die Anzeigevorrichtung gibt die jeweilig für die Entladung des Kondensators notwendige Zeitspanne wieder, welche zwischen dem Maximum des Spannungswertes und bis zu dem Wert nahe 20% des Maximums verstreicht. Sie vergleicht diese Entladezeit mit einem vorgegebenen Soll-Wert. Der Soll-Wert wird dabei mit der beschriebenen Entlade- und Messeinrichtung in folgender Weise bestimmt: Die mit den Widerstandsdrähten 1 und 2 ausgerüstete Messeinrichtung wird in eine repräsentative Probe des Flüssigkeitsgemisches mit dem vorgegebenen prozentualen Grenzwert des in diesem Gemisch enthaltenen Anteiles der Flüssigkeit mit einer niedrigeren Verdampfungstemperatur eingetaucht. Der Widerstandsdraht 1 wird dann mit verschiedenen Energiemengen durch elektrische Kondensatorentladungen kurzzeitig innerhalb von ms bis höchstens einige Sek. erhitzt und die Entladezeiten zwischen einem Spannungswert nahe dem Maximum und nahe 20% des Maximums gemessen sowie derjenige Bereich der Energiemengen ermittelt, innerhalb dessen sich die Entladezeiten merkbar verändern. Diese veränderte Entladezeit wird als Soll-Wert in die Messelektronik eingespeichert und dient bei der späteren Messung in dem Flüssigkeitsgemisch als Vergleichsgrösse. Werden bei späterem Eintauchen der Messeinrichtung in das Flüssigkeitsgemisch bei Kondensatorentladungen über den Widerstandsdraht 1 die in der Messelektronik eingespeicherten Soll-Werte der Entladezeiten erreicht, so ist dies ein Kriterium dafür, dass in dem Flüssigkeitsgemisch der vorgegebene prozentuale Grenzwert des Anteiles der Flüssigkeit mit einer niedrigeren Verdampfungstemperatur erreicht und überschritten ist. Da die Ampulle 4 jeweils mit der Messeinrichtung in die Flüssigkeit eingetaucht wird, nehmen die in der Ampulle befindliche Flüssigkeit und der dort gespannte Widerstandsdraht 2 die Temperatur der umgebenden äusseren Flüssigkeit an. Durch eine Kondensatorentladung über den Widerstandsdraht 2 kann somit der Einfluss der Temperatur des Flüssigkeitsgemisches auf die Entladezeiten ermittelt und in der Messelektronik für die Kondensatorentladungen über den Widerstandsdraht 1 berücksichtigt werden.

In der Messelektronik 6 kann ausserdem ein in der Zeichnung nicht wiedergegebener mit dem Kondensator verbindbarer Konstantwiderstand angeordnet werden in der Weise, dass der Kondensator über einen Wahlschalter über diesen Konstantwiderstand entladen werden kann. Auch bei dieser Entladung werden die Entladezeiten gemessen, so dass Kapazitätsänderungen oder Änderungen der Ladespannungen des Kondensators erfasst und ebenfalls bei der Entladung des Kondensators über den Widerstandsdraht 1 berücksichtigt werden können.

Die Messeinrichtung kann sehr klein ausgebildet werden, so dass sie beispielsweise auch in Vorratsbehälter für die Bremsflüssigkeit eingebaut werden kann, um in Verbindung mit der Messelektronik als Anzeigeeinrichtung für das Überschreiten vorgegebener prozentualer Grenzwerte des von der Bremsflüssigkeit aufgenommenen Wassers verwendet werden zu können.

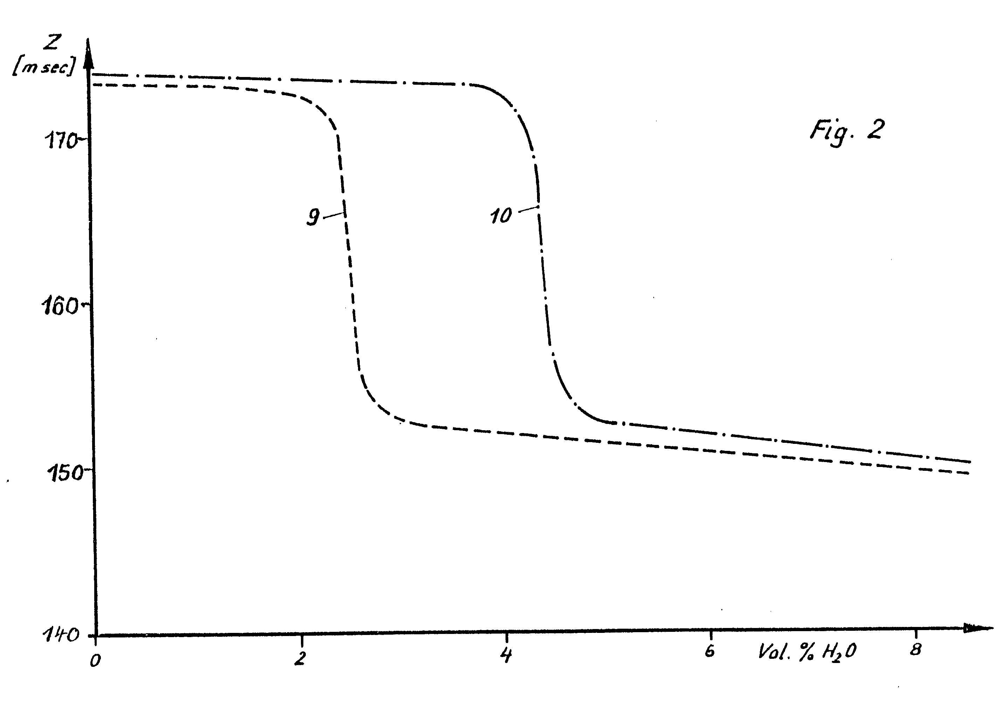

In der graphischen Darstellung gemäss Fig. 2 sind die Entladezeiten eines elektrischen Kondensators gemäss der Fig. 1 in Abhängigkeit von dem Wassergehalt in zwei verschiedenen Hydraulikflüssigkeiten durch die Kurven 9 und 10 wiedergegeben. Man erkennt, dass bei der zu der Kurve 9 gehörigen Flüssigkeit in dem Bereich zwischen 2,2 und 2,3 vol.% Wasser in der Flüssigkeit eine sprunghafte Änderung der Entladezeiten auftritt, während bei der zu der Kurve 10 gehörenden Flüssigkeit diese Änderung etwa zwischen 4,1 und 4,2 vol.% Wasser in der Flüssigkeit gemessen wurde.

Die Kurven zeigen die signifikante Änderung der Entladezeiten, welche sich in bestimmten Bereichen bei nur geringfügiger Änderung des Wassergehaltes in der Hydraulikflüssigkeit ergeben.

Für jeden Grad des Wassergehaltes in der Hydraulikflüssigkeit lassen sich der Fig. 2 entsprechende Kurven aufstellen, wenn dem elektrischen Widerstandsdraht die jeweils geeigneten Energiemengen zugeführt werden.

Ähnliche Kurvenverläufe wie in Fig. 2 ergeben sich auch, wenn andere Messgrössen bei Anwendung des Verfahrens ermittelt werden.

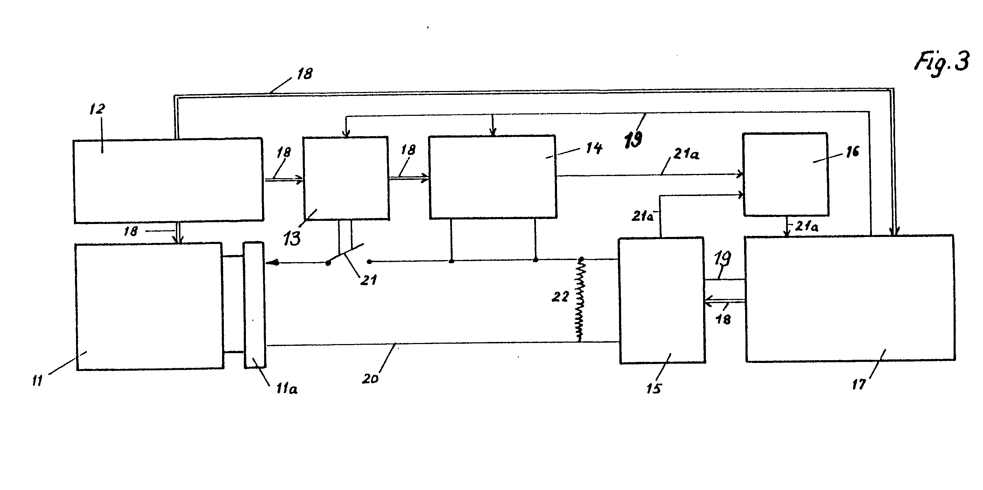

Das in Fig. 3 wiedergegebene Blockschaltbild zeigt eine Anordnung, bei der als Spannungsquelle eine Batterie 11 in Verbindung mit einem Span-

nungsteiler 11a verwendet wird. An die Batterie angeschlossen ist ein Netzteil 12, welches sowohl das Nachladen der Batterie 11 als auch die Stromversorgung einer einstellbaren Zeitschalteinrichtung 13, eines integrierenden Strommessers 14 sowie eines integrierenden Spannungsmessers 15 und der Anzeige- und Steuerelektronik 17 über jeweils mit 18 bezeichnete Versorgungsleitungen übernimmt. Die Elemente 13, 14, 15 sowie 17 und 18 sind ausserdem über Steuerleitungen 19 verbunden.

An den Spannungsteiler 11a ist über Zuleitungen 20 mit dem darin angeordneten Schalter 21 der in das Flüssigkeitsgemisch eintauchbare elektrische Widerstandsdraht 22 angeschlossen. Im Zuge der Zuleitung 20 ist auch der integrierende Strommesser 14 angeordnet, welcher ebenso wie der integrierende Spannungsmesser 15 über Signalleitungen 21a mit einem Multiplikator 16 verbunden ist, der seinerseits über eine weitere Signalleitung 21a mit der Anzeige- und Steuerelektronik 17 in Verbindung steht.

Mittels der Zeitschalteinrichtung 13 bzw. dem Schalter 21 können von der Batterie 11 über den Spannungsteiler 11a dem elektrischen Widerstandsdraht 22 unterschiedliche Energiemengen zugeführt werden, deren Umwandlung in der Anzeige- und Steuerelektronik 17 jeweils angezeigt wird. Durch Veränderung der dem Widerstandsdraht 22 zugeführten Energiemenge kann derjenige Bereich ermittelt werden, innerhalb dessen sich eine sprunghafte Änderung der Energieumwandlung ergibt.

Der Widerstandsdraht 22 entspricht dabei dem Widerstandsdraht 1 in der Fig. 1 und kann ebenso wie in der Darstellung nach Fig. 1 parallel zu einem in einer Ampulle 4 angeordneten weiteren Widerstandsdraht 2 angeordnet sein.

Der in die Flüssigkeit eintauchbare Messsensor, wie er in der Fig. 1 schematisch wiedergegeben ist, kann sehr klein ausgebildet werden, so dass er beispielsweise auch in Vorratsbehälter für die Bremsflüssigkeit von Kraftfahrzeugen eingebaut werden kann, um in Verbindung mit der Messelektronik als Anzeigeeinrichtung für das Überschreiten vorgegebener prozentualer Grenzwerte des von der Bremsflüssigkeit aufgenommenen Wassers verwendet werden zu können.

Da durch die verhältnismässig kurzfristige Beaufschlagung des elektrischen Widerstandsdrahtes mit elektrischer Energie auch nur sehr kurzfristig eine den Draht umhüllende Dampfzone entsteht, welche von der umgebenden Flüssigkeit jedoch wieder absorbiert wird, entstehen keinerlei aufsteigende Dampfblasen, so dass wiederholte Messungen ohne weiteres möglich sind und die Messungen auch in leicht entzündbaren bzw. brennbaren Flüssigkeitsgemischen durchgeführt werden können.

### Patentansprüche

1. Verfahren zur Bestimmung des Erreichens oder Überschreitens eines vorgegebenen prozentualen Grenzwertes des in einem Flüssigkeitsgemisch enthaltenen Anteils mit einer niedrigeren Verdampfungstemperatur als die übrige Flüssigkeit, insbesondere des Wassers in einer Bremsflüssigkeit, mit Hilfe eines elektrischen Widerstandsdrahtes mit positiven Temperaturkoeffizienten, welcher in das Flüssigkeitsgemisch eingetaucht sowie mit einer solchen elektrischen Energiemenge beaufschlagt wird, bei welcher sich die Energieumwandlung in der Probe merkbar verändert, und durch dessen Widerstandsänderung eine Messgrösse beeinflusst wird, dadurch gekennzeichnet, dass zur Eichung der elektrische Widerstandsdraht in einer Probe, die dem zu untersuchenden Flüssigkeitsgemisch entspricht, jedoch den vorgegebenen Grenzwert erreicht bzw. überschreitet, mit verschiedenen definierten Energiemengen durch jeweils einen elektrischen Stromimpuls kurzzeitig innerhalb von ms erhitzt wird und dabei als Messgrösse die zugehörige Zeitspanne für die Einspeisung der definierten Energiemenge in den Widerstandsdraht durch Erfassen der zeitlichen Strom- und/oder Spannungsänderung gemessen sowie die Energiemenge ermittelt wird, bei der sich die Zeitspanne für die Einspeisung zeitlich etwa sprunghaft ändert, und dass zur eigentlichen Messung der in der Probe verwendete Draht oder ein Widerstandsdraht mit den gleichen elektrischen und mechanischen Daten in das zu untersuchende Flüssigkeitsgemisch eingetaucht sowie mit der in der Probe ermittelten Energiemenge, bei welcher sich die Zeitspanne für die Einspeisung zeitlich sprunghaft verändert, beaufschlagt wird und die dabei gemessenen Zeitspannen mit dem in der Probe bei sprunghafter Änderung der Energieeinspeisezeit ermittelten Werte verglichen werden.

2. Verfahren zur Bestimmung des Erreichens oder Überschreitens eines vorgegebenen prozentualen Grenzwertes des in einem Flüssigkeitsgemisch enthaltenen Anteils mit einer niedrigeren Verdampfungstemperatur als die übrige Flüssigkeit, insbesondere des Wassers in einer Bremsflüssigkeit, mit Hilfe eines elektrischen Widerstandsdrahtes mit positivem Temperaturkoeffizienten, welcher in das Flüssigkeitsgemisch eingetaucht sowie mit einer solchen elektrischen Energiemenge beaufschlagt wird, bei welcher sich die Energieumwandlung in der Probe merkbar verändert, und durch dessen Widerstandsänderung eine Messgrösse beeinflusst wird, dadurch gekennzeichnet, dass zur Eichung der elektrische Widerstandsdraht in einer Probe, die dem zu untersuchenden Flüssigkeitsgemisch entspricht, jedoch den vorgegebenen Grenzwert erreicht bzw. überschreitet, während einer jeweils gleichbleibenden bestimmten Zeitspanne im Bereich von ms durch jeweils einen definierten Stromimpuls erhitzt und als Messgrösse die eingespeiste Energiemenge gemessen sowie durch Änderung der Energiemenge der Wert ermittelt wird, bei dem sich die eingespeiste Energiemenge etwa sprunghaft ändert, und dass zur eigentlichen Messung der in der Probe verwendete Draht oder ein Widerstandsdraht mit den gleichen elektrischen und mechanischen Daten in das zu untersuchende

Flüssigkeitsgemisch eingetaucht und in der gleichen Zeitspanne wie in der Probe mit derjenigen Energiemenge beaufschlagt wird, bei welcher sich die eingespeiste Energiemenge in der Probe sprunghaft ändert, und dass diese Energiemenge mit der dem Widerstandsdraht in der Probe bei sprunghafter Änderung zugeführten Energiemenge verglichen wird.

3. Verfahren nach Anspruch 1 oder 2, dadurch gekennzeichnet, dass dem elektrischen Widerstandsdraht die elektrischen Energiemengen bzw. Stromimpulse in der Form eines Auf- oder Endladestromes eines Kondensators oder Speichers zugeführt werden.

4. Verfahren nach Anspruch 3, dadurch gekennzeichnet, dass bei der Zuführung von elektrischen Energiemengen durch Kondensatorentladungen als Messgrösse die Zeitspannen zwischen etwa dem Maximum und etwa 20% des Maximums der Spannung an dem Widerstandsdraht gemessen werden.

5. Verfahren nach einem der Ansprüche 1 bis 3, dadurch gekennzeichnet, dass zur Kompensation von Abweichungen der einspeisbaren Energiemengen, durch Änderungen der Daten der Energiequelle, wie Kapazitätsänderungen oder Spannungsschwankungen, die jeweils für die Messungen in dem Flüssigkeitsgemisch vorgesehenen Energiemengen einem Konstantwiderstand zugeführt werden und die für die Untersuchung des Flüssigkeitsgemisches massgebliche Messgrösse als Ist-Grösse ermittelt und mit der bei der Soll-Menge der eingespeisten Energiemenge ermittelten Messgrösse verglichen wird, und dass die bei der Messung in dem Flüssigkeitsgemisch ermittelte Messgrösse im Verhältnis des Ist-Wertes zum Soll-Wert der Messgrösse bei Beaufschlagung des Konstantwiderstandes korrigiert wird.

6. Verfahren nach einem der vorhergehenden Ansprüche, dadurch gekennzeichnet, dass zur Berücksichtigung der Temperaturänderungen des zu untersuchenden Flüssigkeitsgemisches ein Widerstandsdraht mit den gleichen elektrischen und mechanischen Daten wie der in das Flüssigkeitsgemisch eintauchbare Draht in eine Referenzflüssigkeit eingebracht wird, welche nicht die merkbaren Änderungen der Messgrössen des Gemisches aufweist, dass dem Widerstandsdraht in der Referenzflüssigkeit die jeweils für die Messungen in dem Flüssigkeitsgemisch vorgesehenen Energiemengen bzw. Stromimpulse bei der jeweiligen Betriebstemperatur und bei der Ausgangstemperatur des Flüssigkeitsgemisches zugeführt und die für die Messung im Flüssigkeitsgemisch vorgesehenen Messgrössen ermittelt werden, und dass im Verhältnis dieser Werte der Ist-Wert der in dem Flüssigkeitsgemisch ermittelten Messgrösse korrigiert wird.

7. Vorrichtung zur Durchführung des Verfahrens nach einem der Ansprüche 1 oder 3 bis 6 mit einem in ein Flüssigkeitsgemisch (3) eintauchbaren Widerstandsdraht (1), einer elektrischen Energiequelle zum Beaufschlagen des Widerstandsdrahtes mit Stromimpulsen und einer Messelektronik (6) und Anzeigeeinrichtung, dadurch gekennzeichnet, dass als Energiequelle ein Kondensator vorgesehen ist, dass der Widerstandsdraht (1) in einem Entladekreis des Kondensators angeordnet ist und der Entladekreis mit der Messelektronik (6) und Anzeigeeinrichtung zur Zeitmessung der Energieeinspeisung zur Anzeige der Entladezeiten, zum Vergleich der Entladezeiten mit einem Soll-Wert und zur Anzeige der Differenz zwischen den Entladezeiten und dem Soll-Wert ausgerüstet ist.

8. Vorrichtung zur Durchführung des Verfahrens nach einem der Ansprüche 2 bis 6 mit einem in ein Flüssigkeitsgemisch (3) eintauchbaren Widerstandsdraht (1), einer elektrischen Energiequelle zum Beaufschlagen des Widerstandsdrahtes mit Stromimpulsen und einer Messelektronik (6) und Anzeigeeinrichtung, dadurch gekennzeichnet, dass mit einem elektrischen Kondensator oder Speicher ein Ladekreis verbunden ist, dass der Widerstandsdraht (1) im Ladekreis angeordnet ist, und dass der Ladekreis mit der Messelektronik und Anzeigeeinrichtung zur Messung der Energieeinspeisung, zur Anzeige der in einer vorbestimmten Zeitspanne dem Kondensator bzw. Speicher zugeführte Energiemenge, zum Vergleich der Energiemenge mit einem Soll-Wert und zur Anzeige der Differenz der zugeführten Energiemenge mit dem Soll-Wert vorgesehen ist.

9. Vorrichtung zur Durchführung des Verfahrens nach einem der Ansprüche 2 bis 6 mit einem in ein Flüssigkeitsgemisch eintauchbaren Widerstandsdraht (22), einer in den Widerstandsdraht Stromimpulse einspeisenden elektrischen Energiequelle und einer Messelektronik (17) und Anzeigeeinrichtung, dadurch gekennzeichnet, dass als Energiequelle eine elektrische Batterie (11) vorgesehen ist, dass in einem mit der Batterie verbundenen Stromkreis der Widerstandsdraht (22) angeordnet ist und der Stromkreis mit der Messelektronik (17) zur Vorgabe der Zeitspanne der Energieeinspeisung und zur Anzeige der in einer vorbestimmten Zeitspanne dem Widerstandsdraht zugeführten Energiemenge ausgerüstet sowie mit einer zugeführten Energiemenge mit einem Soll-Wert vergleichenden sowie die Differenz ermittelnden Einrichtung (13 bis 17) verbunden ist.

10. Vorrichtung nach einem der Ansprüche 7 bis 9, dadurch gekennzeichnet, dass parallel zu dem in das Flüssigkeitsgemisch (3) eintauchbaren Widerstandsdraht (1 bis 22) ein Konstantwiderstand angeordnet ist in Verbindung mit einem Wahlschalter zur wahlweisen Verbindung der Energiequelle mit dem Konstantwiderstand oder den in die Flüssigkeit eintauchbaren Widerstandsdraht.

11. Vorrichtung nach einem der Ansprüche 7 bis 10, dadurch gekennzeichnet, dass parallel zu dem in das Flüssigkeitsgemisch (3) eintauchbaren Widerstandsdraht (1 bzw. 22) ein weiterer Widerstandsdraht (2) mit den gleichen elektrischen und mechanischen Daten in einer Ampulle (4) mit einer Flüssigkeit (8) höheren Siedepunktes als der Siedepunkt des zu untersuchenden Flüssigkeitsgemisches angeordnet ist in Verbindung mit einem Wahlschalter zur wahlweisen Verbindung der Energiequelle mit dem in der Ampulle befindlichen oder in die Flüssigkeit eintauchbaren Widerstandsdraht.

## Claims

1. A method for determining when a preset percentage threshold is reached or exceeded by that part of a fluid mixture which has a lower vaporisation temperature than the rest of the fluid, more particularly by the water in a brake fluid, with the aid of an electrical resistance wire having a positive temperature coefficient, which wire is dipped into the fluid mixture and acted upon by a certain quantity of electrical energy, at which quantity the energy conversion in the sample changes noticeably, and a measured value ist influenced by its change in resistance, characterised in that for calibration, the electrical resistance wire, in a sample which corresponds to the fluid mixture to be investigated but which reaches or exceeds the prest threshold, is heated briefly and within milliseconds with different specified quantities of energy by one electrical current pulse in each case, and thereby the related time span for feeding in the specified quantity of energy into the resistance wire is measured as the measured value by detecting the change in current and/or voltage over time, and that quantity of energy for which the feed-in time span changes fairly suddenly over time is determined, and that for the actual measurement the wire used in the sample, or a resistance wire with the same electrical and mechanical data, is dipped into the fluid mixture to be investigated and acted upon by the quantity of energy, determined using the sample, for which the feed-in time span changes suddenly over time, and the time spans thus measured are compared with the reading(s) determined in the sample when there is a sudden change in the energy feed-in time.

2. A method for determining when a preset percentage threshold is reached or exceeded by that part of a fluid mixture which has a lower vaporisation temperature than the rest of the fluid, more particularly by the water in a brake fluid, with the aid of an electrical resistance wire having a positive temperature coefficient, which wire is dipped into the fluid mixture and acted upon by a certain quantity of electrical energy, at which quantity the energy conversion in the sample changes noticeably, and a measured value ist influenced by its change in resistance, characterised in that for calibration, the electrical resistance wire, in a sample which corresponds to the fluid mixture to be investigated but which reaches or exceeds the preset threshold, is heated during a particular constant time span in each case in the region of milliseconds by a specified current pulse in each case and the fed-in quantity of energy is measured as the measured value and the reading at which the fed-in quantity of energy changes fairly suddenly is determined by changing the quantity of energy, and that for the actual measurement the wire used in the sample or a resistance wire with the same electrical and mechanical data is dipped into the fluid mixture to be investigated and is acted upon for the same time span as in the sample with that quantity of energy at which the fed-in quantity of energy in the sample changes suddenly, and that this quantity of energy is compared with the quantity of energy supplied to the resistance wire in the sample when there is a sudden change.

3. A method according to Claim 1 or 2, characterised in that the electrical energy or current pulse is supplied to the electrical resistance wire in the form of a charge or discharge current of a capacitor or store.

4. A method according to Claim 3, characterised in that when the supply of electrical energy is provided by capacitor discharge, the time span between approximately the maximum and approximately twenty percent of the maximum of the voltage at the electrical resistance wire is measured as the measured value.

5. A method according to one of the Claims 1 to 3, characterised in that for compensation of variations of the energy quantity fed-in, due to alterations of the characteristics of the energy source such as capacitance variations or voltage variations, the energy quantity provided each time for the measurement in the fluid mixture is passed to a constant resistance and the determinant measured value for the examination of the fluid mixture is determined as an actual value, which is compared with the measured value determined for the required quantity of energy injected, and that the measured value determined with the measurement in the fluid mixture is corrected in the ratio of the actual to the required values of the measured quantity upon supply to the constant resistance.

6. A method according to one of the preceding Claims, characterised in that in order to take account of the temperature variations of the fluid mixture to be investigated, an electrical resistance wire with the same electrical and mechanical data as the wire which dips into the fluid mixture is placed in a reference fluid which does not have the variation of measured value noticeable for the fluid mixture, that the resistance wire in the reference fluid is supplied with the respective energy quantity or current pulse provided for the measurement in the fluid mixture both at the then working temperatur and at the starting temperature of the fluid mixture and the measured values provided for the measurement in the fluid mixture are determined, and that the actual value determined for the measured value in the fluid mixture is corrected in the ratio of these values.

7. An apparatus for carrying out the process according to one of Claims 1 or 3 to 6, having a resistance wire (1) which can be dipped into a fluid mixture (3), an electrical energy source for supplying the resistance wire eith current pulses, and an electronic measurement device (6) and display arrangement, characterised in that a capacitor is provided as an energy source, that the resistance wire (1) is arranged in a discharge circuit of the capacitor and the discharge circuit is arranged with the electronic measurement device (6) and the display device for measuring the time of energy injection, for display of the discharge times, for the comparison of the discharge times with a desired value, and for the display of the difference between the discharge times and the desired value.

8. Apparatus for carrying out the method according to one of Claims 2 to 6, having a resistance wire (1) which can be dipped in a fluid mixture (3), an electrical energy source for supplying the resistance wire with current pulses and an electrical measurement device (6) and display arrangement, characterised in that a charging circuit is connected with an electrical capacitor or store, that the resistance wire (1) is arranged in the charging circuit and that the charging circuit is arranged with the electronic measuring device and the display arrangement for the measurement of the energy injection, for display of the quantity of energy supplied in a predetermined time period to the capacitor or store, for comparison of the quantity of energy with a desired value, and for display of the difference of the quantity of energy supplied with the desired value.

9. Apparatus for carrying out the method according to one of Claims 2 to 6, having a resistance wire (22) which can be dipped in a fluid mixture, an electrical energy source supplying current pulses to the resistance wire, and an electronic measuring device (17) and a display arrangement, characterised in that an electrical battery (11) is provided as an energy source that the resistance wire (22) is arranged in a current circuit connected with the battery and the current circuit is connected with the electronic measuring device (17) for allowance of the time span for energy injection and for display of the quantity of energy supplied in a predetermined time span to the resistance wire, and is connected also with a device (13 to 17) which compares the quantity of energy supplied with a desired value and determines the difference.

10. Apparatus according to one of the Claims 7 to 9, characterised in that a constant resistance is arranged in parallel with the resistance wire (1 to 22) which can be dipped in the fluid mixture (3), together with a selector switch for selectively connecting the energy source with the constant resistance or with the resistance wire which can be dipped in the fluid.

11. Apparatus according to one of the Claims 7 to 10, characterised in that parallel to the resistance wire (1 or 22) which can be dipped in the fluid mixture (3), a further resistance wire (2) with the same electrical and mechanical data is arranged in an ampoule (4) containing a fluid (8) of higher boiling point than the boiling point of the fluid mixture to be investigated, together with a selector swith for selective connection of the energy source with the resistance wire in the ampoule or with that which can be dipped in the fluid.

**Revendications**

1. Procédé pour déterminer si, dans un mélange de liquides, on a atteint ou dépassé un pourcentage limite prédéterminé pour la fraction qui y est contenue dont la température de vaporisation est inférieure à celle du reste du liquide, en particulier pour l'eau dans un liquide pour frein, à l'aide d'un fil électrique résistant dont le coefficient de température est positif, qui est plongé dans le mélange de liquides, qui est soumis à une quantité d'énergie électrique pour laquelle la transformation de l'énergie varie notablement dans l'échantillon, et dont la variation de résistance influe sur une grandeur de mesure, caractérisé par le fait que, pour l'étalonnage, on chauffe le fil électrique résistant dans un échantillon qui correspond au mélange de liquides à étudier, mais qui atteint ou dépasse la valeur limite prédéterminée, et ce, par diverses quantités d'énergie définies, et au moyens, à chaque fois, d'une impulsion électrique de courte durée inférieure à la milliseconde, cependant que l'on mesure comme grandeur de mesure les intervalles de temps correspondants qui sont nécessaires pour amener la quantité d'énergie définie dans le fil résistant par saisie des variations dans le temps du courant et/ou de la tension, et que l'on détermine aussi la quantité d'énergie pour laquelle l'intervalle de temps pour l'amenée du courant varie dans le temps sensiblement par saut, et par le fait que, pour la mesure proprement dite, on plonge dans le mélange de liquides à étudier le fil utilisé pour l'échantillon ou un fil résistant présentant les mêmes caractéristiques électriques et mécaniques, on le soumet à la quantité d'énergie, obtenue avec l'échantillon, pour laquelle l'intervalle de temps nécessaire pour l'alimentation en courant varie par saut en fonction du temps, et on compare les intervalles de temps ainsi mesurés aux valeurs obtenues dans l'échantillon lors de la variation par saut du temps d'alimentation en énergie.

2. Procédé pour déterminer si, dans un mélange de liquides, on a atteint ou dépassé un pourcentage limite prédéterminé pour la fraction qui y est contenue dont la température de vaporisation est inférieure à celle du reste du liquide, en particulier pour l'eau dans un liquide pour frein, à l'aide d'un fil électrique résistant dont le coefficient de température est positif, qui est plongé dans le mélange de liquides, qui est soumis à une quantité d'énergie électrique pour laquelle la transformation de l'énergie varie notablement dans l'échantillon, et dont la variation de résistance influe sur une grandeur de mesure, caractérisé par le fait que, pour l'étalonnage, le fil électrique résistant est chauffé dans un échantillon qui correspond au mélange de liquides à étudier, mais qui atteint ou dépasse la valeur limite prédéterminée, et ce, pendant un intervalle de temps déterminé, restant le même à chaque fois, et situé dans la zone de la milliseconde, et par une impulsion de courant définie à chaque fois, que l'on mesure comme grandeur de mesure la quantité d'énergie amenée, et que l'on détermine aussi, par variation de la quantité d'énergie, la valeur pour laquelle la quantité d'énergie amenée varie sensiblement par saut, par le fait que, pour la mesure proprement dite, on plonge dans le mélange de liquides à étudier le fil utilisé pour l'échantillon ou un fil résistant présentant les mêmes caractéristiques électriques et mécaniques, on le soumet dans le même intervalle de temps que pour l'échantillon à la quantité d'énergie pour laquelle la quantité d'énergie amenée varie par saut dans l'échantillon, et par le fait que l'on compare cette quantité d'énergie à la quantité d'énergie amenée au fil résistant dans l'échantillon lors de la variation par saut.

3. Procédé selon la revendication 1 ou 2, caractérisé par le fait que les quantités d'énergie ou les impulsions de courant sont amenées au fil électrique résistant sous la forme d'un courant de charge ou de décharge d'un condensateur ou d'un accumulateur.

4. Procédé selon la revendication 3, caractérisé par le fait que, lors de l'amenée de quantités d'énergie électrique par des décharges de condensateurs, on mesure comme grandeur de mesure les intervalles de temps entre environ le maximum de la tension sur le fil résistant et environ 20% de ce maximum.

5. Procédé selon l'une des revendications 1 à 3, caractérisé par le fait que, pour compenser les écarts entre les quantités d'énergie injectables dûs à des variations des données de la source d'énergie, comme des variations de capacité ou des fluctuations de tension, les quantités d'énergie prévues pour chaque mesure dans le mélange de liquides sont amenées à une résistance constante, et la grandeur de mesure qui est déterminante pour l'étude du mélange de liquides est déterminée comme valeur réelle et comparée à la grandeur de mesure déterminée pour la quantité de consigne de la quantité d'énergie injectée, et par le fait que la grandeur de mesure obtenue lors de la mesure dans le mélange de liquides est corrigée en proportion du rapport entre la valeur réelle et la valeur de consigne de la grandeur de mesure lors de l'excitation de la résistance constante.

6. Procédé selon l'une des revendications précédentes, caractérisé par le fait que, pour tenir compte des variations de température du mélange de liquides à étudier, un fil résistant présentant les mêmes caractéristiques électriques et mécaniques que le fil qui peut être plongé dans le mélange de liquides est introduit dans un liquide de référence qui ne présente pas les variations notables des grandeurs de mesure du mélange, par le fait que l'on amène au fil résistant plongé dans le liquide de référence les quantités d'énergie ou, respectivement, les impulsions de courant qui sont prévues pour chaque mesure dans le mélange de liquides pour chaque température de fonctionnement et pour la température de départ du mélange de liquides, et que l'on détermine les grandeurs de mesure prévues pour la mesure dans le mélange de liquides, et par le fait que la valeur réelle de la grandeur de mesure obtenue dans le mélange de liquides est corrigée en proportion de ces valeurs.

7. Dispositif pour la mise en œuvre du procédé selon l'une des revendications 1 ou 3 à 6, comprenant un fil résistant (1) pouvant être plongé dans un mélange de liquides (3), une source d'énergie électrique pour soumettre le fil résistant à des impulsions de courant, et une électronique de mesure (6) et un dispositif d'affichage, caractérisé par le fait qu'un condensateur est prévu comme source d'énergie, que le fil résistant (1) est monté dans un circuit de décharge du condensateur, et que le circuit de décharge est équipé de l'électronique de mesure (6) et du dispositif d'affichage pour mesurer la durée de l'alimentation en énergie, pour indiquer les temps de décharge, pour comparer les temps de décharge à la valeur de consigne, et pour indiquer la différence entre les temps de décharge et la valeur de consigne.

8. Dispositif pour la mise en œuvre du procédé selon l'une des revendications 2 à 6, comprenant un fil résistant (1) pouvant être plongé dans un mélange de liquides (3), une source d'énergie électrique pour soumettre le fil résistant à des impulsions de courant, et une électronique de mesure (6) et un dispositif d'affichage, caractérisé par le fait qu'un circuit de charge est relié à un condensateur électrique ou à un accumulateur, que le fil résistant (1) est monté dans le circuit de charge, et que le circuit de charge est muni de l'électronique de mesure et du dispositif d'affichage pour mesurer l'alimentation en énergie, pour indiquer la quantité d'énergie amenée dans un intervalle de temps prédéterminé au condensateur ou à l'accumulateur, pour comparer la quantité d'énergie à une valeur de consigne, et pour indiquer la différence entre la quantité d'énergie amenée et la valeur de consigne.

9. Dispositif pour la mise en œuvre du procédé selon l'une des revendications 2 à 6, comprenant un fil résistant (22) pouvant être plongé dans un mélange de liquides, une source d'énergie électrique fournissant des impulsions de courant au fil résistant, et une électronique de mesure (17) et un dispositif d'affichage, caractérisé par le fait qu'il est prévu comme source d'énergie une batterie électrique (11), que le fil résistant (22) est monté dans un circuit électrique relié à la batterie, et que le circuit de courant est équipé de l'électronique de mesure (17) pour prédéterminer l'intervalle de temps de l'alimentation en énergie et pour indiquer la quantité d'énergie amenée au fil résistant dans un intervalle de temps prédéterminé, et relié à un dispositif (13 à 17) qui compare la quantité d'énergie amenée à une valeur de consigne, et qui détermine la différence.

10. Dispositif selon l'une des revendications 7 à 9, caractérisé par le fait qu'une résistance constante est montée en parallèle sur le fil résistant (1 ou 22) qui peut être plongé dans le mélange de liquides (3), en liaison avec un commutateur permettant de relier au choix la source d'énergie à la résistance constante ou au fil résistant qui peut être plongé dans le liquide.

11. Dispositif selon l'une des revendications 7 à 10, caractérisé par le fait qu'en parallèle sur le fil résistant (1 ou 22) qui peut être plongé dans le mélange de liquides (3), un autre fil résistant présentant les mêmes caractéristiques électriques et mécaniques est monté dans une ampoule (4) contenant un liquide (8) de point d'ébullition plus élevé que le point d'ébullition du mélange de liquides à étudier, en liaison avec un commutateur permettant de relier au choix la source d'énergie au fil résistant qui se trouve dans l'ampoule ou à celui qui peut être plongé dans le liquide.

Fig. 1

Fig. 2

Fig. 3